Europäisches Patentamt

European Patent Office

Office européen des brevets

(11) Numéro de publication : **0 299 889 B1**

(12) **FASCICULE DE BREVET EUROPEEN**

(45) Date de publication du fascicule du brevet :
**04.03.92 Bulletin 92/10**

(21) Numéro de dépôt : **88420241.7**

(22) Date de dépôt : **11.07.88**

(51) Int. Cl.⁵ : **A61F 2/40**

(54) **Prothèse totale de l'épaule.**

(30) Priorité : **15.07.87 FR 8710495**

(43) Date de publication de la demande :
**18.01.89 Bulletin 89/03**

(45) Mention de la délivrance du brevet :
**04.03.92 Bulletin 92/10**

(84) Etats contractants désignés :
**AT BE CH DE ES FR GB GR IT LI LU NL SE**

(56) Documents cités :
**DE-A- 2 344 569**
**FR-A- 995 762**
**FR-A- 1 107 877**
**US-A- 3 978 528**

(73) Titulaire : **UNIVERSITE DE BOURGOGNE**
**(U.F.R. DE MEDECINE)**
**7 Boulevard Jeanne d'Arc**
**F-21033 Dijon Cédex (FR)**

(72) Inventeur : **Grammont, Paul Marie**
**68 rue Charles Dumont**
**F-21000 Dijon (FR)**
Inventeur : **Trouilloud, Pierre**
**36 rue Charles Dumont**
**F-21000 Dijon (FR)**
Inventeur : **Deries, Xavier**
**15 rue E. Bailly**
**F-21000 Dijon (FR)**

(74) Mandataire : **Maureau, Pierre et al**
**Cabinet GERMAIN & MAUREAU B.P. 3011**
**F-69392 Lyon Cédex 03 (FR)**

Jouve, 18, rue Saint-Denis, 75001 PARIS

## Description

La présente invention concerne une prothèse totale de l'épaule.

Hormis les tumeurs et les nécroses du sujet moyen, la grande majorité des épaules détruites et nécessitant la pose d'une prothèse le sont soit par des fractures comminutives de la tête chez le sujet âgé, dont la coiffe est souvent atteinte, soit par des omarthroses évoluées en luxations supérieures qui sont l'apanage des destructions totales de la coiffe. Or, aucune des prothèses de l'épaule actuellement connues ne donne satisfaction lorsque la coiffe de rotateurs est détruite ou endommagée car elles se contentent de reproduire les surfaces anatomiques de l'articulation, sans pallier l'absence de la coiffe.

Toutes ces prothèses comportent au moins un élément huméral constitué par une tête sphérique destinée à remplacer la tête de l'humérus, tête sphérique solidaire d'une tige destinée à être engagée et fixée dans le canal médullaire de l'humérus, après résection de son extrémité inférieure.

Lorsque la glène, en bon état, n'a pas nécessité la mise en place d'un élément glénoïdien en forme de cupule, la tête sphérique de l'élément huméral prend directement appui contre la glène.

On conçoit aisément que, dans ces conditions, en l'absence de coiffe fonctionnelle et compte tenu de la forte composante ascendante développée par le deltoïde en début d'abduction, l'élément huméral ne peut pas rester fixe et se subluxe vers le haut. Cette subluxation s'accompage généralement d'une usure de la partie supérieure de la glène, engendrant une glénoïdite douloureuse et invalidante qui impose rapidement la pose d'un élément glénoïdien.

Cependant, si cet élément glénoïdien n'est par fortement rétentif, les risques de subluxation vers le haut demeurent et si, par contre, il est fortement rétentif à l'égard d'une subluxation vers le haut, les efforts importants auxquels il est soumis risquent d'entraîner son descellement.

Il peut paraître surprenant, à première vue, que l'on ne puisse pas appliquer à l'articulation de l'épaule des prothèses procédant du même principe que celles utilisées avec succès pour restaurer des articulations de hanches.

Or, la différence provient, semble-t-il, du fait que chez l'homme, qui n'est pas un quadripède, le fémur est porteur tandis que l'humérus ne l'est pas. Il en résulte, en effet, chez l'homme, que lorsque son bras est au repos le long de son corps, le plan tangent à la tête humérale et passant par le point de contact de cette dernière avec la glène est presque vertical de sorte qu'en début d'abduction, la force de traction que le deltoïde doit transmettre à l'humérus, surtout si le bras est chargé, est très élevée. Ce phénomène est d'ailleurs amplifié par le fait que le centre de rotation du bras, confondu avec le centre de la tête humérale, est éloigné de la glène et proche de l'axe de la diaphyse humérale.

C'est probablement à partir de ces observations qu'ont été conçues des prothèses telles que celles connues par les brevets US 3 978 528 et DE 2 344 569 comportant un élément glénoïdien constitué par une pièce mâle sphérique et destiné à être fixé à la glène de l'omoplate et un élément huméral constitué par une cupule sphérique, de même rayon que la pièce mâle sphérique constituant l'élément glénoïdien et solidaire, par sa face externe, d'une tige destinée à être engagée et fixée dans la diaphyse humérale, après résection de l'extrémité supérieure de l'humérus. Cependant, dans ces prothèses connues, pour sa fixation à la glène de l'omoplate, la pièce sphérique de l'élément glénoïdien porte latéralement des piliers d'ancrage qui éloignent considérablement son centre de la glène, ce qui rend plus pénibles les mouvements d'abduction du bras en diminuant la longueur du bras de levier avec lequel le deltoïde agit sur l'humérus. En outre, rien n'est prévu pour permettre un choix de latéralisation de l'articulation en fonction de l'état des muscles et des tendons de cette articulation, ni pour lui procurer une rétention suffisante pour au moins diminuer les risques de luxation.

La présente invention vise à remédier à tous ces inconvénients. A cet effet, dans la prothèse qu'elle concerne et qui est du type précité, la pièce mâle sphérique constituant l'élément glénoïdien est évidée et présente une ouverture s'étendant sur environ deux tiers de sphère et destinée à être engagée et fixée sur la glène, tandis que la cupule sphérique de l'élément huméral s'étend sur environ un tiers de sphère.

Ainsi, le centre d'articulation du bras se trouve fortement déporté en direction de la glène, ce qui favorise considérablement l'abduction du bras en augmentant la longueur du bras de levier avec lequel le deltoïde agit sur l'humérus.

La pièce sphérique évidée de l'élément glénoïdien peut être constituée par une simple calotte sphérique d'épaisseur de deux à cinq millimètres mais l'évidement de cette pièce sphérique peut aussi présenter la forme d'un cylindre dont le fond est destiné à prendre appui, directement ou non, contre la glène après que celle-ci ait été préparée par réalisation d'un cylindre d'encastrement centré sur elle, de même rayon que le logement cylindrique de la pièce sphérique et formé, surtout, en haut, dans le pied de l'apophyse coracoïde et, en bas, dans le pilier de l'omoplate.

La pièce sphérique de l'élément glénoïdien peut être en acier inoxydable, en céramique ou en alliage à base de titane ou de cobalt ou en tout autre produit biocompatible ou en un complexe résultant d'une combinaison de ces différents matériaux.

La pièce sphérique de l'élément glénoïdien et la cupule de l'élément huméral ont avantageusement un diamètre de quarante-deux millimètres.

De préférence, le plan de l'ouverture de la cupule de l'élément huméral fait, avec celui de l'axe de sa tige d'ancrage, un angle compris entre 55° et 70°, angle duquel doit être approchant celui que forme le plan de résection de l'humérus avec l'axe de sa diaphyse.

La fixation à la glène de la sphère de l'élément glénoïdien ainsi que celle de l'élément huméral dans la diaphyse de l'humérus peuvent être améliorées par l'utilisation d'un ciment à os en petite quantité.

L'implantation de l'élément huméral est normalement précédée non seulement de la résection de la tête humérale suivant un plan formant, avec l'axe de sa diaphyse, un angle compris entre 55° et 70°, mais aussi d'une préparation du logement de la tige de cet élément au moyen de deux fraises coniques dont la dimension correspond à la taille de la tige de l'élément huméral considéré.

Au moins la face concave de la cupule de l'élément huméral est en un matériau présentant un faible coefficient de frottement par rapport au matériau constitutif de la face convexe de la pièce sphérique de l'élément glénoïdien.

Suivant une première forme d'exécution simple de l'invention, cet élément huméral est réalisé en polyéthylène de haute densité qui possède l'avantage de présenter un très faible coefficient de frottement à l'égard de la pièce sphérique de l'élément glénoïdien, quel qu'en soit le matériau constitutif.

Suivant une variante d'exécution de l'invention, l'élément huméral est constitué par deux pièces assemblées l'une à l'autre, à savoir un corps en matériau biocompatible comprenant une embase destinée à recevoir la cupule sphérique et solidaire de la tige d'ancrage dans la diaphyse humérale et une cupule présentant une cavité en forme de calotte sphérique, en matériau à faible coefficient de frottement par rapport à celui de la pièce sphérique de l'élément glénoïdien, les faces d'assemblage de la cupule et de l'embase du corps destinée à la recevoir étant agencées pour assurer à la fois leur centrage et leur fixation mutuelle.

De préférence, cet agencement est réalisé de manière à permettre leur fixation mutuelle de manière amovible.

Cet agencement a l'avantage de permettre de prévoir un jeu de cupules différentes présentant des cavités en forme de calotte sphérique d'ampleurs différentes et d'épaisseurs totales différentes.

Le praticien peut ainsi choisir, parmi les cupules de ce jeu, celle correspondant le mieux à la rétention et à la latéralisation désirée en fonction de l'état des muscles et des tendons du patient.

Par exemple, les faces d'assemblage de la cupule et de son embase aménagée sur le corps de l'élément huméral présentent des profils tronconiques complémentaires, respectivement mâle et femelle.

Les profils tronconiques complémentaires des faces d'assemblage de la cupule et de son embase sont avantageusement prolongées, du côté de leur grande base, par une portée cylindrique permettant d'améliorer la fixation de la cupule dans son embase, sans en altérer le caractère amovible.

La cupule est avantageusement en polyéthylène de haute densité et le corps est en acier inox, en titane ou en chrome-cobalt.

De toute façon, l'invention sera bien comprise à l'aide de la description qui suit, en référence au dessin schématique annexé représentant, à titre d'exemple non limitatif, une forme d'exécution de cette prothèse totale de l'épaule :

Figure 1 est une vue de côté en élévation montrant l'articulation d'une épaule droite, avant restauration par cette prothèse :

Figure 2 est une vue de côté en élévation montrant l'élément glénoïdien seul ;

Figure 3 est une vue en coupe axiale d'une forme d'exécution particulière de l'élément glénoïdien ;

Figures 4 est une vue de côté en élévation de l'élément huméral ;

Figure 5 est une vue de côté en élévation, avec coupe partielle, montrant le mode de préparation de la zone glénoïdienne de l'omoplate, en vue de la mise en place de l'élément glénoïdien ;

Figures 6 et 7 sont des vues en coupe axiale de l'extrémité supérieure de l'humérus, après résection, illustrant les deux phases successives de sa préparation, en vue de la mise en place de l'élément huméral ;

Figure 8 est une vue de côté en élévation, avec coupe partielle, montrant l'articulation de figure 1 après mise en place de cette prothèse;

Figure 9 est une vue en coupe axiale d'une variante d'exécution de l'élément huméral, dans le cas où il comporte une cupule amovible ;

Figure 10 est une vue similaire à figure 9 illustrant les effets du choix d'une cupule destinée à augmenter la latéralisation de l'articulation.

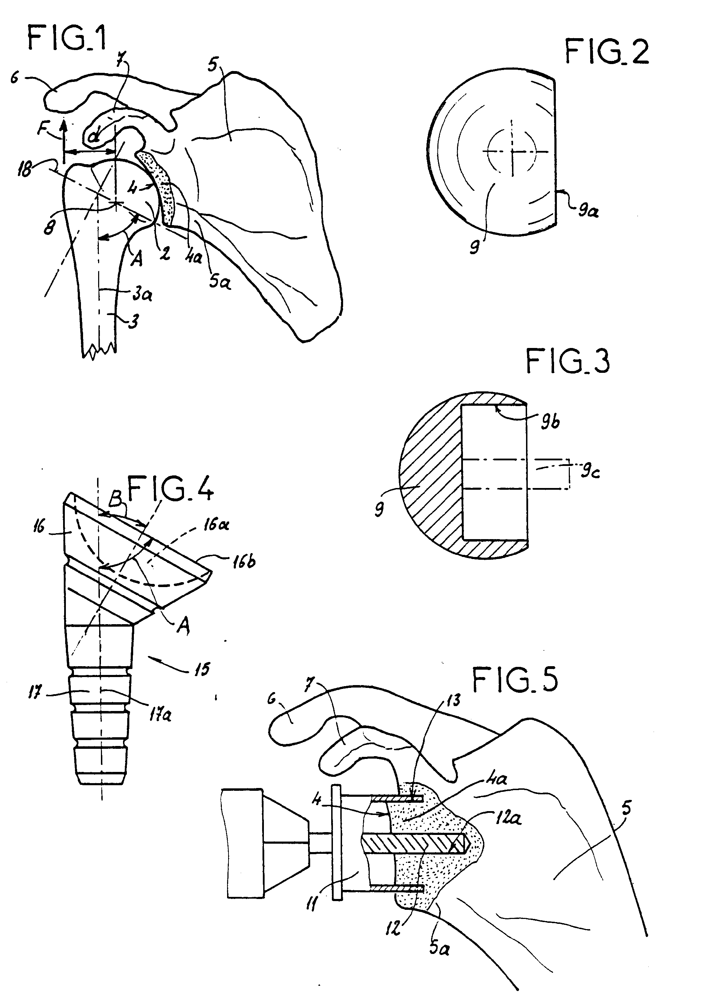

Figure 1 montre une articulation d'épaule droite, vue de face, avec la tête sphérique 2 d'un humérus 3 en appui contre la glène 4 d'une omoplate 5 dont on voit également l'acromion 6 et l'apophyse coracoïde 7.

Comme on peut le voir en examinant cette figure 1, la force F que doit exercer le deltoïde sur l'humérus en début d'abduction, c'est-à-dire en début de mouvement de rotation vers le haut en partant depuis la position normale de repos, le bras pendant à la verticale vers le bas, est très élevée car la distance d séparant la zone d'action du deltoïde sur l'humérus 3 et le centre 8 de la tête sphérique 2 de l'humérus 3 est très petite.

On conçoit aisément que lorsque la coiffe de cette épaule est détruite ou fortement endommagée au point de ne plus assurer la stabilité verticale de la tête 2 de l'humérus 3, la force F tend à provoquer une subluxation vers le haut de cette articulation.

L'objet de la présente invention est donc essentiellement d'allonger la longueur du bras de levier avec lequel le deltoïde agit sur l'humérus 3 ; elle vise par conséquent à augmenter la distance d.

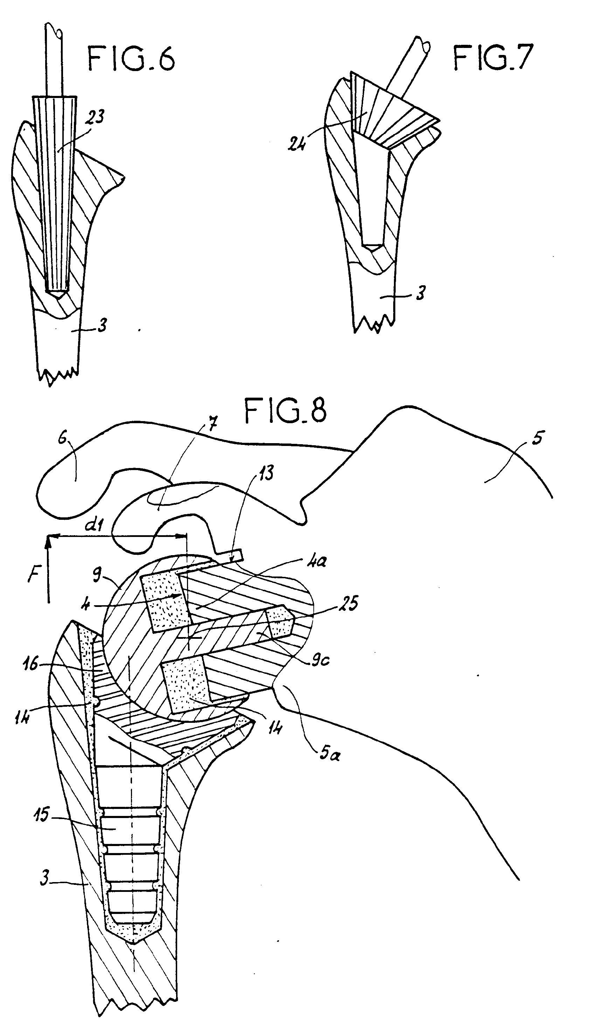

La prothèse totale qu'elle concerne se compose d'un élément glénoïdien constitué essentiellement par une pièce sphérique 9 s'étendant sur environ deux tiers de sphère, représentée sur les figures 2 et 3 et qui est destinée à être fixée sur la glène 4 de l'omoplate 5 en étant fortement engagée sur le col 4a de la glène 4. Pour ce faire, d'une part, la pièce sphérique 9 présente une ouverture 9a, de préférence circulaire et, d'autre part, l'omoplate 5 est préparée en réalisant, comme montré sur la figure 5, à l'aide d'une scie-cloche 11 coaxiale à une mèche de centrage 12, un cylindre d'encastrement 13 entourant la glène 4 et son col 4a. Comme le montre notamment cette figure 5, ce cylindre d'encastrement 13 pénètre fortement, en partie haute, dans le pied de l'apophyse coracoïde 7 et, en partie basse, dans le pilier externe 5a de l'omoplate 5. Ce cylindre d'encastrement 13 permet ensuite l'engagement de l'élément glénoïdien 9 et sa fixation en utilisant, éventuellement, une petite quantité de ciment à os 14.

L'élément glénoïdien 9 peut être réalisé tout simplement à la forme d'une pièce sphérique d'une épaisseur de l'ordre de 2 mm à 5 mm.

Dans une variante d'exécution de cet élément glénoïdien, telle que représentée sur la figure 3, la pièce sphérique 9 présente un évidement cylindrique 9b coaxial à l'ouverture 9a et d'un diamètre lui permettant d'être engagée sur le cylindre d'encastrement 13 préparé à l'aide de la scie-cloche 11. Dans ce cas, il est possible de mettre à profit le perçage 12a réalisé dans le centre de la glène 4 par la mèche de centrage 12 de la scie-cloche 11 pour améliorer la fixation de l'élément glénoïdien 9 à l'omoplate. Il suffit, pour cela, que la pièce sphérique 9 présente une tige d'ancrage 9c coaxiale à l'évidement cylindrique 9b et apte à être engagée dans le perçage 12a, avec ou sans ciment à os.

Cette pièce sphérique 9 constituant l'élément glénoïdien peut être en acier inoxydable, en céramique, en alliage de titane ou de cobalt ou en tout autre matériau biocompatible approprié.

Elle peut aussi être réalisée en un complexe résultant d'une combinaison de ces différents matériaux.

La figure 4 montre l'élément huméral 15 qui comprend, essentiellement, une cupule 16 solidaire, par sa face externe, d'une tige d'ancrage 17. Cette cupule 16 présente un évidement 16a en forme de calotte sphérique s'étendant sur environ un tiers de sphère et de même diamètre que la calotte sphérique 9 de l'élément glénoïdien.

Pour permettre la mise en place de l'élément huméral 15, l'humérus 3 doit être préparé, en premier lieu, par une résection de son extrémité supérieure suivant un plan 18 qui forme, avec l'axe 3a de la diaphyse de l'humérus 3, un angle A compris entre 55° et 70°. Dans l'exemple illustré sur le dessin, cet angle est de 60° car la normale 21 au plan 16b de l'ouverture de l'évidement sphérique 16a de la cupule 16 fait, avec l'axe 17a de sa tige d'ancrage 17, un angle B de 30°.

Le logement de la tige d'ancrage 17 de l'élément huméral 15 est ensuite creusé, comme illustré sur les figures 6 et 7, à l'aide de deux fraises coniques, respectivement 23 et 24. L'élément huméral 15 est alors mis en place et sa fixation peut être améliorée en utilisant une petite quantité de ciment à os 14.

L'élément huméral 15 est avantageusement en un matériau présentant un faible coefficient de frottement par rapport à celui dont est constitué l'élément glénoïdien 9. Lorsque ce dernier est en céramique d'alumine ou en acier inoxydable, l'élément huméral 15 est avantageusement en polyéthylène haute densité.

La figure 8 montre l'épaule de figure 1 après mise en place de la prothèse totale de l'invention et l'examen comparatif des figures 1 à 8 permet de constater que la distance d1 séparant, sur la figure 8, le centre 25 de l'élément glénoïdien 9 de la ligne d'action du deltoïde sur l'humérus 3, c'est-à-dire de la force F, est bien supérieure à la valeur d illustrée sur la figure 1 car la constitution et le mode de montage de cette prothèse permettent de déplacer considérablement le centre de l'articulation (centre 25) en direction de la glène 4 de l'omoplate 5 et, ce de fait, d'augmenter de façon importante la longueur du bras de levier avec lequel le deltoïde agit sur l'humérus 3.

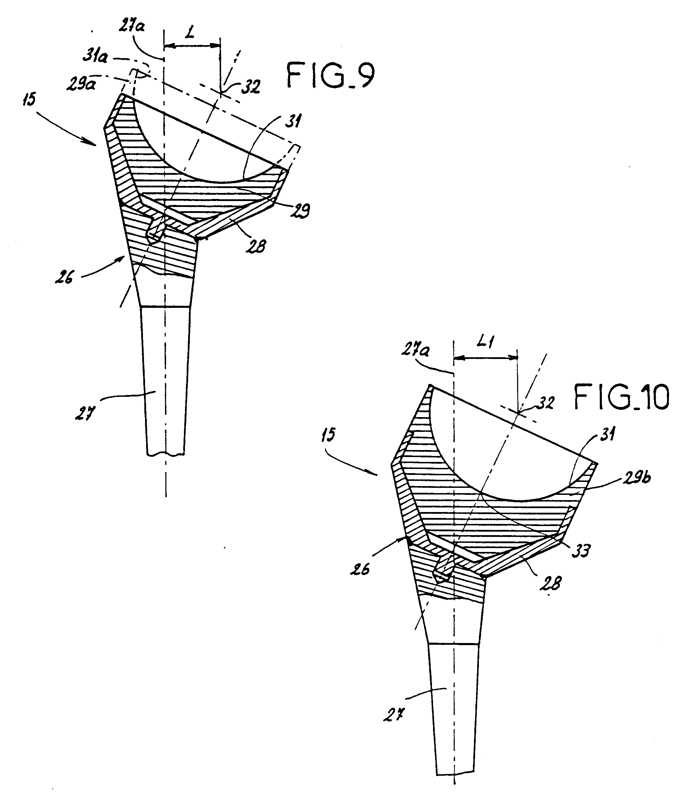

Les figures 9 et 10 illustrent une variante d'exécution de l'élément huméral 15 permettant d'adapter cette prothèse à l'état de l'articulation du patient, notamment en fonction de ses distensions éventuelles de muscles et de ligaments.

Dans cet exemple, l'élément huméral 15 est réalisé en deux parties assemblées l'une à l'autre, à savoir un corps 26 et une cupule 29. Le corps 26, qui est réalisé en un matériau biocompatible tel qu'en acier inox, titane ou chrome-cobalt, comporte une tige 27 d'ancrage dans la diaphyse humérale surmontée d'une embase 28 destinée à recevoir et maintenir une cupule 29 présentant une cavité 31 en forme de calotte sphérique et dont le matériau constitutif est choisi de manière à présenter un faible coefficient de frottement par rapport à la face externe sphérique de l'élément glénoïdien 9. Un matériau convenant parfaitement à la réalisation de cette cupule 29 est le polyéthylène haute densité.

Pour que l'assemblage de la cupule 29 à son embase 28 soit réalisé de manière à permettre son centrage et sa fixation correcte, leurs faces d'assemblage sont avantageusement conformées en tronc de cône, respectivement mâle et femelle, emboîtables l'un dans l'autre, l'embase 28 servant alors de berceau à la cupule 29.

Les parties tronconiques complémentaires de la cupule 29 et de son embase 28 sont avantageusement prolongées, du côté de leur grande base, par une portée cylindrique améliorant, par coincement, la tenue de la cupule 29 dans son embase 28, sans en altérer le caractère amovible.

Cet agencement confère à cet assemblage l'amovibilité propre à permettre le choix et la mise en place d'une cupule 29 au dernier moment par le praticien, un jeu de cupules 29, 29a ou 29b différentes pouvant être alors mises à la disposition de ce dernier.

La figure 9 montre, en traits pleins, l'utilisation d'une cupule 29 à faible valeur rétentive, utilisable lorsque les muscles et les tendons du patient ne présentent pratiquement aucune distension. En effet, dans ce cas, il n'est pas nécessaire d'augmenter la distance L séparant l'axe 27a de la tige d'ancrage 27 du centre de la calotte sphérique 31 de la cupule 29, ni d'augmenter l'ampleur de la calotte sphérique que constitue la cavité 31 en rapprochant le plan de son ouverture du plan équatorial contenant son centre 32.

Sur la figure 9, on a montré, en traits mixtes, une cupule 29a dont le centre 32 est confondu avec celui de la cupule 29 mais dont le plan d'ouverture de la cavité 31a est très proche du plan équatorial contenant le centre 32 de cette cavité.

Dans l'exemple illustré sur la figure 10, la cupule 29b assemblée au corps 26 présente la même rétention que la cupule 29a de l'exemple illustré en traits mixtes sur la figure 9. Par contre, la distance L1 séparant l'axe 27a de la tige d'ancrage 27 et le centre 32 de la cavité en forme de calotte sphérique 31 de la cupule 29b est nettement supérieure à la distance correspondante L de l'exemple précédent. Cette différence est due à une augmentation de l'épaisseur totale de la cupule 29, qui a eu pour effet d'augmenter la distance séparant le fond 33 de la cavité 31 et la petite base du berceau tronconique de l'embase 28.

Comme le montrent les exemples qui viennent d'être décrits, cet assemblage amovible de la cupule 29 au corps 26 et la présence d'un jeu de capules différentes 29a à 29b permettent au praticien d'adapter au dernier moment la géométrie de cette prothèse à l'état de l'articulation du patient qui la reçoit.

## Revendications

1. Prothèse totale de l'épaule, du type comportant un élément glénoïdien (9) constitué par une pièce mâle sphérique et destiné à être fixé à la glène (4) de l'omoplate et un élément huméral (15) constitué par une cupule sphérique (16), de même rayon que la pièce mâle sphérique constituant l'élément glénoïdien (9) et solidaire, par sa face externe, d'une tige (17) destinée à être engagée et fixée dans la diaphyse humérale, après résection de l'extrémité supérieure de l'humérus (3), **caractérisée en ce que** la pièce mâle sphérique constituant l'élément glénoïdien (9) est évidée et présente une ouverture (9a) s'étendant sur environ deux tiers de sphère et destinée à être engagée et fixée sur la glène (4), tandis que la cupule sphérique (16) de l'élément huméral (15) s'étend sur environ un tiers de sphère.

2. Prothèse selon la revendication 1, caractérisée en ce que l'élément glénoïdien (9) est constitué par une ample calotte sphérique et présente une épaisseur de deux millimètres.

3. Prothèse selon la revendication 1, caractérisée en ce que l'élément glénoïdien (9) présente un évidement cylindrique (9b) coaxial à l'ouverture (9a) et dont le fond est destiné à prendre appui contre la glène (4) après que celle-ci ait été préparée par réalisation d'un cylindre d'encastrement (13) centré sur elle, de même rayon que le logement cylindrique (9b) de la calotte sphérique (9) et formé, surtout, en haut, dans le pied de l'apophyse coracoïde (7) et, en bas, dans le pilier (5a) de l'omoplate (5).

4. Prothèse selon l'une quelconque des revendications précédentes, caractérisée en ce que l'élément glénoïdien (9) est en acier inoxydable, en céramique, en alliage à base de titane ou de cobalt ou en tout autre matériau biocompatible ou en un complexe résultant d'une combinaison de ces différents matériaux.

5. Prothèse selon l'une quelconque des revendications précédentes, caractérisée en ce qu'au moins la face concave de la cavité (16a) de la cupule (16) de l'élément huméral (15) est en un matériau présentant un faible coefficient de frottement par rapport au matériau constitutif de la face convexe de la pièce sphérique (9) de l'élément glénoïdien.

6. Prothèse selon la revendication 5, caractérisée en ce que cet élément huméral (15) est réalisé entièrement en polyéthylène de haute densité.

7. Prothèse selon la revendication 5, caractérisé en ce que l'élément huméral (15) est constitué par deux pièces assemblées l'une à l'autre, à savoir un corps (26) en matériau biocompatible comprenant une embase (28) destinée à recevoir la cupule sphérique (29) et solidaire de la tige (27) d'ancrage dans la diaphyse humérale et une cupule (29, 29a, 29b) présentant une cavité (31) en forme de calotte sphérique, en matériau à faible coefficient de frottement par rapport à celui de la pièce sphérique (9) de l'élément glénoïdien, les faces d'assemblage de la cupule (29, 29a, 29b) et de l'embase (28) du corps (26) destinée à la recevoir étant agencées pour assurer à la fois leur centrage et leur fixation mutuelle.

8. Prothèse selon la revendication 7, caractérisée en ce que l'assemblage entre la cupule (29, 29a, 29b) et son embase (28) est amovible.

9. Prothèse selon la revendication 8, caractérisé en ce qu'il est prévu un jeu de cupules différentes (29, 29a, 29b) présentant des cavités (31) en forme de calotte sphérique d'ampleurs différentes et d'épaisseurs totales différentes.

10. Prothèse selon l'une quelconque des revendications 7 à 9, caractérisée en ce que les faces d'assemblage de la cupule (29, 29a, 29b) et de son embase (28) aménagée sur le corps (26) de l'élément huméral (15) présentent des profils tronconiques complémentaires, respectivement mâle et femelle.

11. Prothèse selon la revendication 10, caractérisée en ce que les profils complémentaires des faces d'assemblage de la cupule (29, 29a, 29b) et de son embase (28) sont prolongées, du côté de leur grande base, par une portée cylindrique permettant d'améliorer la fixation de la cupule (29, 29a, 29b) dans son embase (28), sans en altérer le caractère amovible.

12. Prothèse selon l'une quelconque des revendications 7 à 11, caractérisée en ce que la cupule (29, 29a, 29b) est en polyéthylène de haute densité et le corps (26) est en acier inox, titane ou chrome-cobalt.

13. Prothèse selon l'une quelconque des revendications précédentes, caractérisée en ce que le diamètre de la calotte sphérique de l'élément glénoïdien (9) et celui de l'évidement (16a) de la cupule (16) de l'élément huméral est de l'ordre de quarante-deux millimètres.

14. Prothèse selon l'une quelconque des revendications précédentes, caractérisée en ce que le plan de l'ouverture (16b) de la cupule (16) de l'élément huméral (15) fait, avec l'axe (17a) de sa tige d'ancrage (17), un angle (A) compris entre 55° et 70°, angle duquel doit être approchant celui que forme le plan de résection (18) de l'humérus (3) avec l'axe (3a) de sa diaphyse.

## Patentansprüche

1. Totale Schultergelenk-Endoprothese, des Typs beinhaltend ein glenoides Element (9), gebildet durch ein vorstehendes Kugelstück und dazu bestimmt, an der Gelenkpfanne (4) des Schulterblattes befestigt zu werden, sowie ein Oberarmelement (15), gebildet durch einen Kugelnapf (16) gleichen Durchmessers wie das vorstehende Kugelstück, das das glenoide Element (9) bildet, wobei es mittels seiner Außenfläche fest an einer Stange (17) sitzt, die dazu bestimmt ist, in die Oberarmdiaphyse gesteckt und darin befestigt zu werden, nach Resektion des oberen Endes des Oberarmknochens (3), **dadurch gekennzeichnet**, daß das das glenoide Element bildende vorstehende Kugelstück (9) ausgespart ist und eine sich über zwei Drittel der Kugel erstreckende Öffnung (9) aufweist und dazu bestimmt ist, auf der Gelenkpfanne (4) angeordnet und befestigt zu werden, während der Kugelnapf (16) des Oberarmelementes (15) sich über etwa ein Kugeldrittel erstreckt.

2. Endoprothese nach Anspruch 1, dadurch gekennzeichnet, daß das glenoide Element (9) aus einer großräumigen Kugelkalotte gebildet ist und eine Dicke von 2 mm aufweist.

3. Endoprothese nach Anspruch 1, dadurch gekennzeichnet, daß das glenoide Element (9) eine zu der Öffnung (9a) koaxial zylindrische Aussparung (9b) aufweist, deren Grund dazu bestimmt ist, sich gegen die Gelenkpfanne (4) abzustützen, nachdem diese durch Bildung eines auf ihr zentrierten Einbauzylinders (13) entsprechend präpariert ist, der den gleichen Radius wie die zylindrische Aussparung (9b) der Kugelkalotte (9) hat und der im wesentlichen, oben, im Fuß der coracoiden Apophyse (7) und unten in dem Pfeiler (5a) des Schulterblattes (5) gebildet ist.

4. Endoprothese nach einem der vorhergehenden Ansprüche, dadurch gekennzeichnet, daß das glenoide Element (9) aus rostfreiem Stahl, aus Keramik, aus einer Legierung auf der Basis Titan oder Kobalt oder jedem anderen biokompatiblen Material oder einem Komplex, resultierend aus einer Kombination dieser verschiedenen Materialien hergestellt ist.

5. Endoprothese nach einem der vorhergehenden Ansprüche, dadurch gekennzeichnet, daß zumindest die konkave Fläche der Höhlung (16a) des Napfes (16) des Oberarmelementes (15) aus einem Material mit geringem Reibkoeffizienten bezüglich des die konvexe Fläche des Kugelstückes (9) des glenoiden Elementes bildenden Materiales besteht.

6. Endoprothese nach Anspruch 5, dadurch gekennzeichnet, daß das Oberarmelement (15) in seiner Gesamtheit aus einem Hochdruckpolyäthylen besteht.

7. Endoprothese nach Anspruch 5, dadurch gekennzeichnet, daß das Oberarmelement (15) aus zwei miteinander montierten Stücken besteht, nämlich einem Körper (26) aus einem biokompatiblen Material, beinhaltend ein Basisstück (28) zur Aufnahme des Kugelnapfes (29) und fest verbunden mit der Stange (27) zur Verankerung in der Oberarmdiaphyse, sowie einen Napf (29, 29a, 29b), der eine Höhlung (31) in Form einer Kugelkalotte aus einem Material mit geringem Reibkoeffizienten bezüglich des Materiales des Kugelstückes (9) des glenoiden Elementes aufweist, wobei die Verbindungsflächen des Napfes (29, 29a, 29b) und des Basisstückes (28) des Körpers (26) zu dessen Aufnahme so ausgebildet sind, daß sie gleichzeitig ihre Zentrierung und ihre gegenseitige Festsetzung gewährleisten.

8. Endoprothese nach Anspruch 7, dadurch gekennzeichnet, daß die Verbindung zwischen dem Napf (29, 29a, 29b) und dem Basisstück (28) lösbar ausgebildet ist.

9. Endoprothese nach Anspruch 8, dadurch gekennzeichnet, daß ein Satz unterschiedlicher Näpfe (29, 29a, 29b) vorgesehen ist, der Höhlungen (31) in Kugelkalottenform unterschiedlicher Weite und unterschiedlicher Gesamtdicken aufweist.

10. Endoprothese nach einem der Ansprüche 7 - 9, dadurch gekennzeichnet, daß die Verbindungsflächen des Napfes (29, 29a, 29b) und des auf dem Körper (26) des Oberarmelementes (15) angeordneten Basisstückes (28) jeweils vorspringende und aufnehmende komplementäre Kegelstumpfprofile aufweisen.

11. Endoprothese nach Anspruch 10, dadurch gekennzeichnet, daß die komplementären Profile der Verbindungsflächen des Napfes (29, 29a, 29b) und seines Basisstückes (28) auf der Seite ihrer großen Basis durch eine zylindrische Lagerfläche verlängert sind, die die Verbesserung der Befestigung des Napfes (29, 29a, 29b) an seinem Basisstück (28) zu verbessern erlaubt, ohne den lösbaren Charakter zu verändern.

12. Endoprothese nach einem der Ansprüche 7 - 11, dadurch gekennzeichnet, daß der Napf (29, 29a, 29b) aus Hochdruckpolyäthylen besteht und der Körper (26) aus rostfreiem Stahl, Titan oder Chrom-Kobalt besteht.

13. Endoprothese nach einem der vorhergehenden Ansprüche, dadurch geikennzeichnet, daß der Durchmesser der Kugelkalotte des glenoiden Elementes (9) und derjenige der Aussparung (16a) des Napfes (16) des Oberarmelementes in der Größenordnung von 42 mm liegen.

14. Endoprothese nach einem der vorhergehenden Ansprüche, dadurch gekennzeichnet, daß die Ebene der Öffnung (16b) des Napfes (16) des Oberarmelementes (15) mit der Achse (17a) in seiner Verankerungsstange (17) einen Winkel A zwischen 55° und 70° bildet, einen Winkel, dem angenähert sein muß derjenige, den die Reseiktionsebene (16) des Oberarmknochens (3) mit der Achse (3a) seiner Diaphyse bildet.

**Claims**

1. A complete shoulder prosthesis, of the type including a glenoid element (9) constituted by a spherical male component and intended to be fixed to the glenoid cavity (4) of the scapula and a humeral element (15) constituted by a spherical cupule (16) having the same radius as the spherical male component constituting the glenoid element (9) and rigidly connected by its external face to a rod (17) intended to be engaged and fixed in the humeral diaphysis after resection of the upper end of the humerus (3), characterised in that the spherical male portion constituting the glenoid element (9) is hollowed but and has an opening (9a) extending over about two thirds of a sphere and intended to be engaged and fixed on the glenoid cavity (4), whilst the spherical cupule (16) of the humeral element (15) extends over about one third of a sphere.

2. A prosthesis according to Claim 1, characterised in that the glenoid element (9) is constituted by a large spherical dome and has a thickness of 2mm.

3. A prosthesis according to Claim 1, characterised in that the glenoid element (9) has a cylindrical hollow (9b) co-axial with the opening (9a) and of which the bottom is intended to bear against the glenoid cavity (4) after the latter has been prepared by forming using a fitting cylinder (13) centred thereon, of the same radius as the cylindrical housing (9b) of the spherical dome (9) and formed, mainly at the top in the foot of the coracoid process (7) and at the bottom in the column (5a) of the scapula (5).

4. A prosthesis according to any one of the preceding claims, characterised in that the glenoid element (9) is of stainless steel, ceramic, a titanium or cobalt based alloy or of any other bio-compatible material or of a complex resulting from a combination of these different materials.

5. A prosthesis according to any one of the preceding claims, characterised in that at least the concave face of the cavity (16a) of the cupule (16) of the humeral element (15) is of a material having a low coefficient of friction with respect to the material constituting the convex face of the spherical part (9) of the glenoid element.

6. A prosthesis according to Claim 5, characterised in that this humeral element (15) is formed entirely of high density polyethylene.

7. A prosthesis according to Claim 5, characterised in that the humeral element (15) is constituted by two parts assembled together, i.e. a body 26 of bio-compatible material comprising a base (28) intended to receive the spherical cupule (29) and rigidly connected to the rod (27) for anchoring in the humeral diaphysis and a cupule (29, 29a, 29b) having a cavity (31) in the form of a spherical dome, of material having a low co-efficient of friction with respect to that of the spherical part (9) of the glenoid element, the assembly faces of the cupule (29, 29a, 29b) and the base (28) of the body (26) intended to receive it being arranged to ensure both their centering and their mutual securement.

8. A prosthesis according to Claim 7, characterised in that the assembly between the cupule (29, 29a, 29b) and its base (28) is removable.

9. A prosthesis according to Claim 8, characterised in that a set of different cupules (29, 29a, 29b) is provided having spherically domed cavities of different sizes and different total thicknesses.

10. A prosthesis according to any one of Claims 7 to 9, characterised in the assembly faces of the cupule (29, 29a, 29b) and its base (28) formed on the body (26) of the humeral element (15) have respectively male and female complementary truncated cone profiles.

11. A prosthesis according to Claim 10, characterised in that the complementary profiles of the assembly faces of the cupule (29, 29a, 29b) and its base (28) are extended, from the side of their large base, by a cylindrical bearing surface permitting the fixing of the cupule (29, 29a, 29b) on its base (28) to be improved, without impairing its removable character.

12. A prosthesis according to any one of claims 7 to 11, characterised in that the cupule (29, 29a, 29b) is of high density polyethylene and the body (26) is of stainless steel, titanium or cobalt chrome.

13. A prosthesis according to any one of the preceding claims, characterised in that the diameter of the spherical dome of the glenoid element (9) and that of the cavity (16a) of the cupule (16) of the humeral element is of the order of 42mm.

14. A prosthesis according to any one of the preceding claims, characterised in that the plane of the opening (16b) of the cupule (16) of the humeral element (15) makes, with the axis (17a) of its anchoring rod (17), an angle A between 55° and 70°, which angle must approach that which forms the re-section plane (18) of the humerus (3) with the axis (3a) of is diaphysis.

FIG.1

FIG.2

FIG.3

FIG.4

FIG.5

FIG.6

*23*

*3*

FIG.7

*24*

*3*

FIG.8

*6*

*7*

*5*

*13*

*d1*

*F*

*9*

*4a*

*4*

*25*

*16*

*9c*

*14*

*14*

*5a*

*15*

*3*

10

FIG_9

FIG_10